# EUROPEAN PATENT APPLICATION

(11) **EP 1 312 676 A1**
(43) Date of publication of application: **21.05.2003**
(21) Application number: 01956995.3
(22) Date of filing: 21.08.2001
(51) Int. Cl.: C12N 15/12, A61K 35/76, A61K 38/00, A61K 39/395, A61K 45/00, C07K 16/40, C12N 9/64, C12Q 1/37

(54) **NOVEL PROTEASE GENE**

(30) Priority: 23.08.2000 JP 2000252503
(71) Applicant: DAIICHI PHARMACEUTICAL CO., LTD., Chuo-ku, Tokyo 103-8234 (JP)
(72) Inventor: YOKOTA, Hiroshi DAIICHI PHARMACEUTICAL CO., LTD., Edogawa-ku, Tokyo 134-8630 (JP); ARAI, Yasuko DAIICHI PHARMACEUTICAL CO., LTD., Edogawa-ku, Tokyo 134-8630 (JP); WADA, Naoya DAIICHI PHARMACEUTICAL CO., LTD., Edogawa-ku, Tokyo 134-8630 (JP); INOUE, Tatsuya DAIICHI PHARMACEUTICAL CO., LTD., Edogawa-ku, Tokyo 134-8630 (JP); SAEKI, Yoshiyuki DAIICHI PHARMACEUTICAL CO., LTD., Edogawa-ku, Tokyo 134-8630 (JP)
(74) Representative: Krauss, Jan B., Dr.
(86) International application number: JP0107147
(87) International publication number: WO02016610

(57) **Abstract**

It is intended to provide a novel diagnostic means and pharmaceutical compositions by finding out and providing a novel neurolysin-like substance in humans, analyzing the function, localization, etc. of the novel neurolysin-like substance in humans, clarifying the role of this substance in vivo and thus controlling its function. A novel neurolysin-like substance having an activity of degrading a synthetic substrate Mcc-Pro-Leu-Gly-Pro-D-Lys(DNP)-OH is found out.

## Description

### TECHNICAL FIELD

The present invention relates generally to a novel human protease and a gene encoding the protease, and more particularly to a novel human protease highly homologous to porcine, rabbit, and rat neurolysins.

### BACKGROUND ART

Neurolysin (EC 3.4.24.16) found in pig, rabbit, and rat, is also called "neurotensin-degrading neutral metalloendopeptidase", and is known to inactivate neurotensin by cleaving the peptide at the position of Pro¹⁰-Tyr¹¹ bond. Neurotensin is a brain/intestinal peptide having vasodilatory, hypotensive, hypoglycemic, and analgesic actions.

Neurolysin exists ubiquitously in various tissues of rat. In addition, it was reported that the distribution of neurolysin in the brain was the same as that of the neurotensin receptor. However, in the primary cultured neurons derived from mouse embryos expressing neurolysin, the population expressing neurolysin is quite different from the population in which neurotensin receptor is found. Therefore, it is considered that neurolysin exists in the vicinity of neurotensin receptor, degrades neurotensin and interferes with the signal transduction of neurotensin via its receptor. Purification and analysis of neurolysin are described in "Methods in Enzymology 248, 593-614, 1995" in detail. However, human neurolysin has not been reported so far.

One of the subjects of the present invention is to find out and provide a human neurolysin-like substance. Another subject of the present invention is to analyze the function and the localization of the human neurolysin-like substance, to elucidate a role of the substance in the body, and to provide a novel tool for the diagnosis and a novel pharmaceutical composition by controlling the function of the substance.

### DISCLOSURE OF THE INVENTION

On the way to zealous investigation for solving the above problems, a protease motif search was carried out in a cDNA library derived from the human brain to find a novel protease gene, and its whole-length cDNA was determined to provide a human neurolysin-like gene.

More specifically, the present invention provides:
1. a peptide selected from a group consisting of:
   (i) a peptide consisting of the amino acid sequence of SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH, and
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH;
2. a peptide having an amino acid sequence that consists of four consecutive amino acids or more of the amino acid sequence of SEQ ID NO:1;
3. a polynucleotide encoding a peptide selected from a group consisting of the following peptides, and a complementary strand thereto:
   (i) a peptide consisting of the amino acid sequence of SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate.
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH, and
   (v) a peptide having an amino acid sequence that consists of four consecutive amino acids or more of the amino acid sequence of SEQ ID NO:1;
4. a polynucleotide consisting of the base sequence of SEQ ID NO:2 or a complementary strand thereto;
5. a polynucleotide according to above 3 or 4, wherein the polynucleotide hybridizes under stringent conditions;
6. a polynucleotide consisting of a base sequence that consists of at least twelve consecutive bases of the base sequence of the polynucleotide encoding the amino acid sequence of SEQ ID NO:1 or a complementary strand thereto, or a complementary strand thereto;
7. a recombinant vector comprising a polynucleotide encoding a peptide selected from the group consisting of those from (i) to (v) or a polynucleotide selected from a group consisting of those from (vi) to (viii):
   (i) a peptide consisting of the amino acid sequence of SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
   (v) a peptide having an amino acid sequence that consists of four consecutive amino acids or more of the amino acid sequence of SEQ ID NO:1,
   (vi) a polynucleotide consisting of the base sequence of SEQ ID NO:2, or a complementary strand thereto,
   (vii) a polynucleotide that hybridizes to a polynucleotide encoding the peptide described in (i), (ii), (iii), (iv) or (v) under stringent conditions, and
   (viii) a polynucleotide consisting of a base sequence that consists of at least twelve consecutive bases of the base sequence of the polynucleotide encoding the amino acid sequence of SEQ ID NO:1 or a complementary strand thereto;
8. plasmid pDEST17-hNZMP (FERM BP-7269);
9. a transformant transformed with a recombinant vector comprising a polynucleotide encoding a peptide selected from the group consisting of those from(i) to (v), a recombinant vector comprising a polynucleotide selected from the group consisting of those from (vi) to (viii), or the plasmid (ix):
   (i) a peptide consisting of the amino acid sequence of SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
   (v) a peptide having an amino acid sequence that consists of four consecutive amino acids or more of the amino acid sequence of SEQ ID NO:1,
   (vi) a polynucleotide consisting of the base sequence of SEQ ID NO:2 or a complementary strand thereto,
   (vii) a polynucleotide that hybridizes to a polynucleotide encoding the peptide described in (i), (ii), (iii), (iv) or (v) under stringent conditions,
   (viii) a polynucleotide consisting of a base sequence that consists of at least twelve consecutive bases of the base sequence of the polynucleotide encoding the amino acid sequence of SEQ ID NO:1 or a complementary strand thereto, and
   (ix) Plasmid pDEST17-hNZMP (FERM BP-7269);
10. a method for producing a peptide of above 1 or 2, comprising a step of culturing a transformant of above 9;
11. an antibody that immunologically recognizes a peptide selected from a group consisting of:
   (i) a peptide consisting of the amino acid sequence of SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH, and
   (v) a peptide having an amino acid sequence that consists of four consecutive amino acids or more of the amino acid sequence of SEQ ID NO:1;
12. an antibody of above 11, wherein the antibody has at least a function to decrease the activity of degrading synthetic substrate Mcc-Pro-Leu-Gly-Pro-D-Lys(DNP)-OH;
13. a method for identifying a compound that interacts with a peptide selected from a group consisting from (i) to (v) to decrease or increase its activity, and/or a compound that interacts with a polynucleotide selected from a group consisting from (vi) to (viii) to decrease or increase its expression, wherein the method uses at least one selected from a group consisting of the peptide, the polynucleotide, a recombinant vector comprising the polynucleotide, plasmid pDEST17-hNZMP (FERM BP-7269), a transformant obtained by using the recombinant vector or the plasmid, and an antibody that immunologically recognizes the peptide:
   (i) a peptide consisting of the amino acid sequence show by SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
   (v) a peptide having an amino acid sequence that consists of four consecutive amino acids or more of the amino acid sequence of SEQ ID NO:1,
   (vi) a polynucleotide consisting of a base sequence of SEQ ID NO:2,
   (vii) a polynucleotide encoding each of the peptides described in (i)-(v) or a polynucleotide that hybridizes to the polynucleotide under stringent conditions, and
   (viii) a polynucleotide consisting of a base sequence that consists of at least twelve consecutive bases of the base sequence of the polynucleotide encoding the amino acid sequence of SEQ ID NO:1 or a complementary strand thereto;
14. a method for identifying a compound according to above 13, wherein the method includes contacting a peptide with a compound to be screened under conditions that permit the interaction of the compound with the peptide, evaluating the interaction of the compound, and detecting a signal given by the interaction of the compound with the peptide, followed by determining whether the compound increases or decreases the activity by the interaction of the compound with the peptide;
15. a method for identifying a compound according to above 13 or 14, wherein the method includes contacting a transformant with a compound to be screened, introducing a system using a signal and/or marker capable of detecting the expression of the peptide, detecting the presence of the signal, followed by determining the presence or absence of an increase or decrease of the expression of the peptide;
16. a compound identified by a method according to any of above 13 to 15;
17. a compound of above 16, which interacts with the peptide to decrease or increase its activity;
18. a compound of above 16, which interacts with the polynucleotide to decrease or increase its expression;
19. a pharmaceutical composition comprising any one selected from the group consisting of the following peptides (i)-(v), the following polynucleotides (vi)-(viii), recombinant vectors containing the polynucleotides, plasmid pDEST17-hNZMP (FERM BP-7269), transformants obtained by using the recombinant vectors or the plasmids, and antibodies that immunologically recognize the peptides:
   (i) a peptide consisting of the amino acid sequence show by SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
   (v) a peptide having an amino acid sequence that consists of four consecutive amino acids or more of the amino acid sequence of SEQ ID NO:1,
   (vi) a polynucleotide consisting of a base sequence of SEQ ID NO:2,
   (vii) a polynucleotide encoding each of the peptides described in (i)-(v) or a polynucleotide that hybridizes to the polynucleotide under stringent conditions, and
   (viii) a polynucleotide consisting of a base sequence that consists of at least twelve consecutive bases of the base sequence of the polynucleotide encoding the amino acid sequence of SEQ ID NO:1 or a complementary strand thereto;
20. a use of the pharmaceutical composition of above 19 as a medicament for preventing/treating diseases that relates to the peptide consisting of an amino acid sequence of SEQ ID NO:1;
21. a method for preventing/treating diseases that relates to the peptide consisting of an amino acid sequence of SEQ ID NO:1, using the pharmaceutical composition of above 19; and
22. a method for diagnosing diseases that relate to the expression or activity of a peptide selected from a group consisting of the following peptides (i)-(v), comprising analyzing for a peptide selected from a group consisting of the following peptides (i)-(v) or a polynucleotide selected from the group consisting of the following polynucleotides (vi)-(viii) as a marker:
   (i) a peptide consisting of the amino acid sequence show by SEQ ID NO:1,
   (ii) a peptide comprising the peptide described in (i),
   (iii) a peptide that has an amino acid sequence homologous to the peptide described in (i) at about 40% or more, and has an activity of degrading synthetic substrate
      Mcc-Pro-Leu-Gly-Pro-D-Lys(DNP)-OH,
   (iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
   (v) a peptide having an amino acid sequence that consists of four consecutive amino acids or more of the amino acid sequence of SEQ ID NO:1,
   (vi) a polynucleotide consisting of a base sequence of SEQ ID NO:2,
   (vii) a polynucleotide encoding each of the peptides described in (i)-(v) or a polynucleotide that hybridizes to the polynucleotide under stringent conditions, and
   (viii) a polynucleotide consisting of a base sequence that consists of at least twelve consecutive bases of the base sequence of the polynucleotide encoding the amino acid sequence of SEQ ID NO:1 or a complementary strand thereto.

### BRIEF DESCRIPTION OF DRAWINGS

Fig. 1 illustrates the structure of the gene (KIAA1226) found in the cDNA library derived from the human brain, the N-terminal side structure (pBS/hNZMP-N#7) that is considered to be deleted from the gene, and the constructed whole-length human NZMP structure (pBS/hNZMP#1).
Fig. 2 illustrates the expression of human NZMP in *Escherichia coli*. The arrowhead indicates the position of the band of NZMP.
Fig. 3 illustrates the result of the solubility test of human NZMP. The arrowhead indicates the position of the band of NZMP.
Fig. 4 illustrates the result of test for the culture-condition to obtain a water-soluble human NZMP. The arrowhead indicates the position of the band of NZMP.
Fig. 5 illustrates the expression of a water-soluble human NZMP in *Escherichia coli*. The arrowhead indicates the position of the band of NZMP.
Fig. 6 illustrates the results of the production of a large amount of human NZMP by expressing in *Escherichia coli* and of the confirmation of its solubility in water. The arrowhead indicates the position of the band of NZMP.
Figs. 7A-7D illustrate the results of test for enzyme activity of human NZMP expressed in *Escherichia coli*. Fig. 7A, Fig.7B, Fig.7C, and Fig.7D indicate the enzyme activity assayed at 0 h, 1 h, 2 h, and 3 h after induction of the gene expression, respectively.
Fig. 8 illustrates the enzyme activity of 1.563 µg/ml of human NZMP during induction of the gene expression.

### BEST MODE FOR CARRYING OUT THE INVENTION

### Novel neurolysin-like substance

A novel neurolysin-like substance provided by the present invention is a peptide encoded by a novel gene that was obtained by carrying out a protease motif search in a long-chain cDNA library derived from the human brain (DNA Research 4, 53-59, 1997) and by selecting cDNA (KIAA1226), followed by determining its whole-length cDNA. The novel gene is highly homologous to genes of pig, rabbit, and rat neurolysins, and its gene product has a function to degrade synthetic substrate
Mcc-Pro-Leu-Gly-Pro-D-Lys(DNP)-OH. The gene product consists of 681 amino acids, and has a zinc metallopeptidase motif from 471st Thr to 480th Met. Thus, it was found that the novel gene encodes a novel neurolysin-type zinc metallopeptidase. This novel gene is called "NZMP (novel zinc metallopeptidase) gene", and its gene product is called 'NZMP' hereafter.

NZMP gene is highly expressed in the corpus callosum of human. In addition, a search for a natural intracerebral substrate by searching in the enzyme substrate database based on the amino acid sequence of the above synthetic substrate led to the discovery of a dopamine D5 receptor having an amino acid sequence of Pro-Leu-Gly-Pro as a substrate for NZMP (Nature 350, 614-619, 1991). Amino acid sequence Pro-Leu-Gly-Pro is the binding site region for a dopamine D5 receptor to its ligand, so that NZMP according to the present invention was assumed to play an important role in the signal transduction system in the brain. Outside the brain, it was found that ICAM-1 (J. Immunol. 147, 2777-2786, 1991) has an amino acid sequence of Pro-Leu-Gly-Pro in the binding site region to its ligand LFA-1 (Proc. Natl. Acad. Sci. U.S.A. 95, 4134-4139, 1998), and the relation between the novel neurolysin-like substance according to the present invention and ICAM-1 was also found.

### Peptide

The present invention provides the above-described NZMP and a peptide derived thereof. As used herein, the 'peptide' means a substance comprising two or more amino acids linked to each other by a peptide bond, and includes protein, polypeptide, and oligopeptide and so on.

NZMP according to the present invention is a peptide consisting of the amino acid sequence of SEQ ID NO:1. The peptide according to the present invention can be a peptide comprising the peptide that consists of the amino acid sequence of SEQ ID NO:1.

The peptide according to the present invention can be a peptide that is homologous to the peptide having the amino acid sequence of SEQ ID NO:1 at about 40% or more, preferably about 70% or more, more preferably about 80% or more, much more preferably about 90% or more, most preferably about 95% or more, and has at least an activity of degrading synthetic substrate Mcc-Pro-Leu-Gly-Pro-D-Lys(DNP)-OH. The homology between amino acid sequences can be determined by a well-known method such as a method for directly determining the amino acid sequences and a method for determining base sequences of cDNAs followed by deducing amino acid sequences encoded by them. The peptide according to the present invention can be selected from homologous peptides using, as an indicator, at least an activity of degrading the synthetic substrate Mcc-Pro-Leu-Gly-Pro-D-Lys(DNP)-OH (Methods in Enzymology 248, 593-614, 1995).

The present invention also includes peptides having a partial sequence of the peptide consisting of an amino acid sequence of SEQ ID NO:1. These can be utilized, for example, as reagents, standard substances, antigens, and so on. The peptide according to the present invention consists of 4 to 8 amino acids or more, preferably 10 amino acids or more, more preferably 12 amino acids or more, much more preferably 15 amino acids or more, and is preferably the peptide that can be immunologically recognized.

The peptides according to the present invention can be used as reagents, standard substances, and antigens for preparing a specific antibody against NZMP solely or in a form linked to a carrier as described later (e.g., keyhole limpets hemocyanin or egg albumin). The present invention also provides a peptide bound to other protein or substance as such.

In addition, based on thus specified peptide, a peptide consisting of an amino acid sequence that was modified by mutation such as deletion, substitution, addition or insertion of one amino acid or more, for example 1 to 100 amino acid(s), preferably 1-30 amino acid(s), more preferably 1-20 amino acid(s), much more preferably 1-10 amino acid(s), in particular preferably one to a few amino acids is also provided using, as an indicator, at least the activity to degrade the synthetic substrate
Mcc-Pro-Leu-Gly-Pro-D-Lys(DNP)-OH. Deletion, substitution, addition and insertion can be carried out by a well-known method, such as the site specific mutation-inducing method, the gene homologous recombination method, the primer elongation method, and the polymerase chain reaction (PCR) method, used solely or by a combination of these methods according to books such as Sambrook et al. ed. "Molecular Cloning, A Laboratory Manual 2nd ed." Cold Spring Harbor Laboratory, 1989, Masami Muramatsu ed. "Labo-manual Idensi Kougaku"] Maruzen Co., Ltd., 1988, and Ehrlich HA, ed. "PCR technology. Principles and applications for DNA amplification" Stockton Press 1989, or by modifying those, for example, using Ulmer's technique (Science 219, 666, 1983).

When introducing such mutation, in view of preventing a change of the fundamental properties (such as the physical properties, activity, or immunological activity) of the peptide, mutual substitution among, for example, homologous amino acids (polar amino acids, non-polar amino acids, hydrophobic amino acids, hydrophilic amino acids, positively charged amino acids, negatively charged amino acids, aromatic amino acids, and so on) can be carried out.

The present invention also provides a peptide having an NZMP activity comparable to that of the peptide consisting of the amino acid sequence of SEQ ID NO:1 and its minimal active unit (region or domain) as well as a peptide whose activity intensity or substrate specificity was modified. These are useful as an NZMP activity-like substance or NZMP antagonist, or for screening a substance controlling an NZMP activity, and so on.

In addition, it is easy for those skilled in the art to add other peptide such as alkaline phosphatase, β-galactosidase, immunoglobulin Fc fragment (e.g., IgG), or FLAG-tag directly, or indirectly via linker peptide or the like, using a genetic engineering technique or other technique, to N-terminal side or C-terminal side in order to facilitate the detection and/or purification of the peptide according to the present invention, or in order to add other function(s). Therefore, the present invention also provides peptides bound to other substances.

### Polynucleotide

The present invention also provides a polynucleotide encoding the above peptide according to the present invention, or a complementary strand thereto. For example, the polynucleotide according to the present invention means a polynucleotide encoding the peptide consisting of the amino acid sequence of SEQ ID NO:1, or a complementary strand thereto. The polynucleotide is preferably the polynucleotide consisting of the base sequence of SEQ ID NO:2.

The present invention also provides a polynucleotide that hybridizes to a region corresponding to the above polynucleotide according to the present invention or the complementary strand thereto, preferably the polynucleotide consisting of a base sequence of SEQ ID NO:2 or a complementary strand thereto under stringent conditions. Hybridization can be carried out using a well-known method, Sambrook et al. ed. "Molecular Cloning, A Laboratory Manual 2nd ed." Cold Spring Harbor Laboratory (1989). These polynucleotides can be those that hybridize to the objective polynucleotide, in particular the polynucleotide consisting of the base sequence of SEQ ID NO: 2 or a complementary strand thereto, and need not have a complementary sequence. For example, the polynucleotide can be a polynucleotide homologous to the polynucleotide consisting of the base sequence of SEQ ID NO:2 or a complementary strand thereto at about 40% or more, for example about 70% or more, preferably about 80% or more, more preferably about 90% or more, much more preferably about 95% or more. The polynucleotide according to the present invention includes a polynucleotide, oligonucleotide and a complementary strand thereto consisting of a sequence that consists of consecutive 10 nucleotides or more, preferably 15 nucleotides or more, more preferably 20 nucleotides or more, which corresponds to the region of the specified base sequence.

These polynucleotides are useful, for example, for producing the peptide according to the present invention, as a probe or primer for detecting a nucleic acid encoding NZMP, for example, its gene or mRNA, or as an antisense oligonucleotide for controlling the gene expression. Therefore, the nucleotide according to the present invention includes not only the translated region but also ones corresponding to the untranslated region. For example, it is preferable to use a base sequence characteristic to NZMP in order to specifically inhibit the expression of NZMP by an antisense oligonucleotide.

The base sequence of a polynucleotide encoding NZMP or a peptide having a similar activity can be determined, for example, by confirming the protein expressed by using a known protein-expressing system, followed by selection using its physiological properties, at least an activity of degrading synthetic substrate Mcc-Pro-Leu-Gly-Pro-D-Lys(DNP)-OH as an indicator. For the protein-expressing system, for example, a cell-free protein-expressing system such as a ribosome system derived from, for example, wheat germ or rabbit reticulocyte (Nature 179, 160-161, 1957) can be used.

### Recombinant vector

A recombinant vector can be obtained by inserting the polynucleotide according to the present invention into an adequate DNA vector. The DNA vector may be a naturally existing one and also may be one that lacks a part of its DNA other than that necessary for replication (e.g., vectors derived from Col E1, vectors derived from a lambda phage). As a method for inserting the polynucleotide according to the present invention into the above-described DNA vector, the well-known methods can be employed. For example, a method can be used, wherein an appropriate restriction enzyme is chosen for treating a DNA to cleave it at a specific site, and then, the DNA is mixed with the DNA used as a vector treated in the same way, followed by ligating again with a ligase.

For the present invention, using a plasmid DNA as the DNA vector, as described in Examples described later, plasmid pDEST17-hNZMP comprising NZMP gene according to the present invention was prepared. The plasmid was deposited in National Institute of Bioscience and Human-Technology, Agency of Industrial Science and technology, Ministry of International Trade and Industry with a deposition number of FERM BP-7269 on August 10, 2000.

### Transformant

The present invention can provide a peptide consisting of NZMP and a derivative thereof according to the present invention by the gene recombination technique using a well-known host such as *Escherichia coli*, yeast, *Bacillus subtilis*, insect cells, and animal cells as well as the above cell-free protein-expressing system.

The transformation of a host can be carried out by applying a well-known means using, for example, plasmid, chromosome, or virus as a replicon. A preferable system is exemplified by the method for integrating the gene into the chromosome, in view of achieving stability of the gene. However, an autonomous replication system using a plasmid can be conveniently used. A vector is selected considering the kind of the host selected, and consists of the constitutional elements of the desired DNA sequence to be expressed and a DNA sequence possessing information relating to replication and regulation. A promoter, a ribosome-binding site, a terminator, a signal sequence, an enhancer, and the like can be used in combination, wherein the combination is selected depending on whether the host is a prokaryote or eukaryote. Although *Escherichia coli* was used for Examples in the present specification, a host is not limited thereto.

A transformant can be cultured under the well-known conditions suitable for each host. The peptide is produced by the sub-cultivation or batch culture, using the amount of the transformant in the medium, or the activity of NZMP or the derivative peptide thereof that was expressed by the transformant, particularly at least the activity to degrade synthetic substrate Mcc-Pro-Leu-Gly-Pro-D-Lys(DNP)-OH, as an indicator.

### Collecting NZMP or derivative thereof

A peptide consisting of NZMP or a derivative peptide thereof can be purified/collected by a method, such as a gel filtration chromatography, an ion column chromatography, an affinity chromatography, and the like, in combination, or by fractionation means on the basis of a difference in solubility using ammonium sulfate, alcohol, and the like, from a medium in which the above transformant is cultured, using, as an indicator, at least the activity to degrade synthetic substrate
Mcc-Pro-Leu-Gly-Pro-D-Lys(DNP)-OH. More preferably used is a method, wherein the peptides are specifically adsorbed and collected by using polyclonal antibodies or monoclonal antibodies, which are prepared against the peptides based on the information of their amino acid sequences.

### Antibody

An antibody is prepared by selecting an antigenic determinant of a peptide consisting of NZMP according to the present invention or a derivative peptide thereof. The antigen can be NZMP or a fragment thereof, and consists of at least 8 amino acids, preferably at least 10 amino acids, more preferably at least 12 amino acids, much more preferably 15 amino acids or more. In order to prepare the antibody specific to NZMP, a region consisting of the amino acid sequence intrinsic to the above-described polypeptide or peptide is desirably used. The amino acid sequence is not necessarily homologous to the amino acid sequence of SEQ ID NO:1, but is preferably a site exposed outside of a stereo-structure of the protein. In such a case, it is preferable that the amino acid sequence of the exposed site is consecutive in the exposed site, if it is discrete in its primary structure. Any antibody can be used as long as the antibody immunologically binds or recognizes NZMP or a derivative peptide thereof. The presence or absence of the binding or recognition can be determined by a well-known antigen-antibody binding reaction.

In order to produce an antibody, a well-known method for antibody production can be employed. For example, the antibody is obtained by administration of NZMP or the derivative peptide thereof according to the present invention to an animal in the presence or absence of an adjuvant with or without linking such to a carrier so as to induce humoral immunity and/or cell-mediated immunity. Any carrier can be used as long as it is not harmful to the host. For example, cellulose, a polymerized amino acid, albumin, and the like are exemplified, but not limited thereto. As an animal used for immunization, a mouse, rat, rabbit, goat, horse, and so on, is preferably used.

A polyclonal antibody can be obtained from serum of an animal subjected to the above-described immunological means by a well-known method for collecting antibodies. A preferable means is exemplified by immunoaffinity chromatography.

A monoclonal antibody can be produced by collecting antibody-producing cells (for example, a lymphocyte derived from a spleen or a lymph node) from the animal subjected to the above-described immunological means, followed by introducing a well-known transformation means with a permanently proliferating cell (for example, myeloma strain such as P3/X63 - Ag8 cells.) For example, the antibody-producing cells are fused with the permanent proliferating cells by a well-known method to prepare hybridomas. Then, the hybridomas are subjected to cloning, followed by selecting ones producing the antibody that recognizes specifically the above-described polypeptide or peptide to collect the antibody from a culture solution of the hybridoma.

The polyclonal antibody or the monoclonal antibody thus obtained, which recognizes and binds to NZMP or a derivative peptide, can be utilized as an antibody for purification, a reagent, a labeling marker and so on. In particular, an antibody capable of suppressing the activity of NZMP (e.g., an antibody capable of decreasing the enzymatic activity of NZMP) can be used for controlling the activity of NZMP according to the present invention, so that it is useful for treating and/or preventing diseases related to NZMP.

### Identifying, research and screening tool

NZMP or the derivative peptide thereof according to the present invention, the polynucleotide and the complementary strand thereto according to the present invention, the cell transformed based on the information concerning the amino acid and base sequences, the recombinant vector containing a polynucleotide according to the present invention, the plasmid according to the present invention, and the antibody immunologically recognizing NZMP or the derivative peptide thereof, thus obtained, provide an effective means for identifying and/or screening an inhibitor and/or activator for NZMP or the derivative peptide thereof, when using them solely or in combination. For example, the screening of an antagonist by the drug design based on the stereo-structure of a peptide, the screening of an expression-regulating agent by gene level using a protein-synthesizing system, and the screening of an antibody-recognizing substance using an antibody, can be carried out by using one of well-known drug-screening systems.

NZMP or the derivative peptide thereof according to the present invention permits identifying a compound that enhances or inhibits the function of NZMP or the derivative peptide thereof according to the present invention, by selecting conditions that permit interaction of a compound intended to screen to the peptide, and introducing a system using a signal and/or marker that permits detecting the presence or absence of any interaction (such interaction is related to the second component that permits providing a detectable signal corresponding to the interaction of the compound to the peptide), followed by detecting the presence or absence of the signal and/or marker.

A transformant according to the present invention permits identifying a compound that increases or decreases the expression of NZMP or the derivative peptide thereof according to the present invention, by contacting the transformant with a compound intended to screen, introducing a system using a signal and/or marker that permits detecting the presence or absence of the expression of NZMP or the derivative peptide thereof according to the present invention, followed by detecting the presence or absence of the signal and/or the marker.

### Compound

A compound identified by the above means can be used as a candidate compound for an inhibitor, an antagonist, or an activator with respect to the activity of NZMP or the derivative peptide thereof. In addition, it can also be used as an inhibitor, an antagonist, or an activator for the expression of NZMP or the derivative peptide thereof. Such a candidate compound is expected to have an effect of preventing/treating diseases related with NZMP.

### Pharmaceutical composition

The compound thus selected can be used in a pharmaceutical composition by selecting ones having both biological usefulness and low toxicity. NZMP or the derivative peptide thereof according to the present invention, a polynucleotide and a complementary strand thereto according to the present invention, a transformed cell based on the information concerning these amino acid and base sequences, a vector containing a polynucleotide according to the present invention, a plasmid according to the present invention, and an antibody immunologically recognizing a peptide consisting of NZMP or a derivative thereof, can be used as a disease-diagnosing means such as diagnostic marker, reagent, and so on. They can be used as a pharmaceutical means such as medicament utilizing the function to inhibit, to antagonize, or to activate the activity and/or expression of NZMP. In other words, the present invention provides a pharmaceutical composition comprising at least one species thereof, when using them solely or in combination with each other. For the pharmaceutical preparation, it is sufficient to use a well-known means for their preparation according to each of the object such as peptide, protein, polynucleotide, or antibody.

### Diagnostic means

NZMP or the derivative peptide thereof according to the present invention or a polynucleotide according to the present invention is useful as a means for diagnosing diseases related with the expression or activity of NZMP. For example, the diagnosis is carried out by determining the amount of the corresponding nucleic acid molecule utilizing the interaction or reactivity with a nucleic acid molecule encoding NZMP, and/or by determining the distribution of NZMP or the derivative peptide thereof in an individual living body, and/or by determining the amount of NZMP or the derivative peptide thereof in a specimen derived from an individual. In other words, the assay is carried out using NZMP as a diagnosis marker. For the assay, for example, a well-known antigen-antibody reaction system, the enzyme reaction system, and the PCR system can be used. 'Means' used herein means a method and/or medium that is used to achieve a purpose. Namely, 'Means' includes a method for the diagnosis and a reagent kit for the diagnosis. The reagent kit for the diagnosis or the reagent for the diagnosis comprises at least one selected from a group consisting of a peptide according to the present invention, a polynucleotide according to the present invention, and an antibody according to the present invention. In addition, it can contain a buffer solution and/or a marker, and so on that is necessary for the measurement to carry out the diagnosis.

### Examples

The present invention will be illustrated more concretely with the following examples, but is not limited thereto.

### Example 1

### Identification of human NZMP

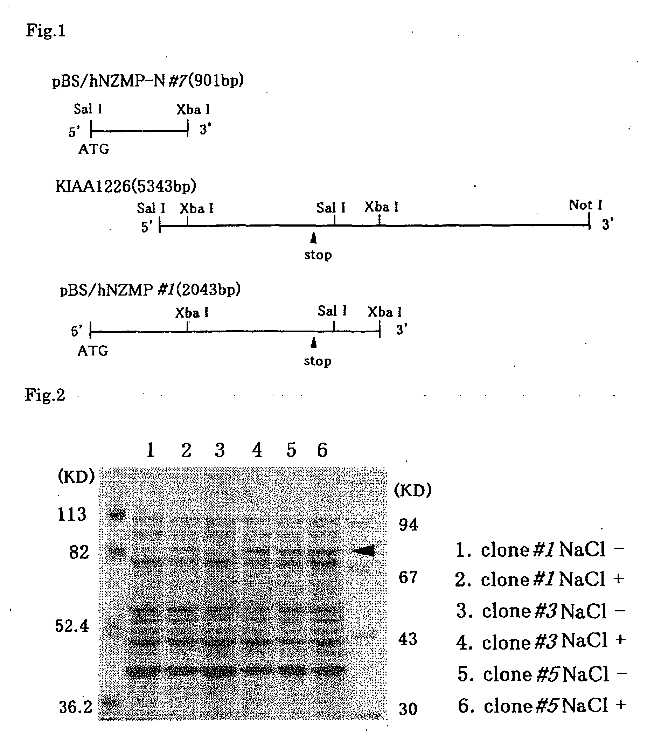

A cDNA (KIAA1226) having a protease motif was found by carrying out the protease motif search by the bioinformatics using a cDNA library derived from the human brain, which was constructed by Kazusa DNA Research Institute (DNA Research 4, 53-59, 1997). The cDNA (KIAA1226) obtained was considered to have a deletion of a part of the N terminus. Therefore, in order to determine the whole-length cDNA, the sequence of the N terminus was estimated based on a genome-DNA database (Fig. 1), and primers for PCR were designed to carry out cloning of the deleted part by the RT-PCR method. 5' -side primer was designed based on a base sequence estimated from the database, while 3'-side primer was designed based on XbaI site region in the upstream of KIAA1226 with taking account of the recombination. The base sequences of these primers are shown below:

### Primers

Pr-K1226-S01 (SEQ ID NO:3)

5'-AGGTCGACATGACGTTAGGAAGAGAA-3'

SalI

Pr-K1226-AS04 (SEQ ID NO:4)

5'-TCATCTAGAAAGGCTGTTACGC-3'

XbaI

The reverse transcription reaction was carried out using the above primers and a Super SCRIPT II kit (Gibco BRL, Div. of Life Technologies Inc.) as follows: Reaction mixture 1 (described below) was heated at 70°C for 10 min, and was chilled on ice, followed by adding 4 µl of 5 x First Strand buffer, 2µl of 0.1M DTT, and 1 µl of 10 mM dNTP Mix. Then, 1 µl of Super SCRIPT II (200 units/µl) was added to carry out a reaction at room temperature for 10 min, and at 42°C for 50 min, finally at 70°C for 15 min.

| Reaction mixture 1 | |
|---|---|
| Oligo (dT) primer (0.5 µg/µl) | 1 µl |
| mRNA derived from human brain(1 µg/µl) | 0.2 µl |
| H₂O | 10.8 µl |
| Total | 12 µl |

Then, 25 µl of Mix1 and 25 µl of Mix2 (both shown below) were mixed, and the PCR was carried out using an Expand High Fidelity PCR System (Boehringer Ingelheim Corp.) with the cycle shown below.

| Mix1 | |
|---|---|
| dNTP Mix (1.25 mM) | 5 µl |
| Pr-K1226-S01 (26.6 pmol/µl) | 0.8 µl |
| Pr-K1226-AS04 (35.2 pmol/µl) | 0.6 µl |
| cDNA | 2 µl |
| H₂O | 16.6 µl |
| | 25 µl |

| Mix2 | |
|---|---|
| 10 x Expand HF buffer | 5 µl |
| Expand HF (3.5 u/µl) | 0.8 µl |
| H₂O | 19.2 µl |
| | 25 µl |

| Cycle | |
|---|---|
| Pre-step | 94°C 3 min |
| Step 1 | 94°C 30 sec |
| Step 2 | 60°C 30 sec |
| Step 3 | 72°C 1 min |
| | (30 cycles from Step 1 to Step 3) |
| Post-step | 72°C 3 min |

The PCR product obtained was subjected to treatment for making blunt ends and for phosphorylation using a BKL kit (Takara Bio Inc.). Reaction mixture 2 described below was incubated at 37°C for 10 min, followed by applying to a micropure-EZ filter cup to centrifuge at 15,000 rpm for 30 sec. A part of the filtrated PCR product was ligated to pBluescript (SK-) vector (BAP-treated after SmaI digestion), and was used to transform a competent cell (JM109).

| Reaction mixture 2 | |
|---|---|
| PCR product solution | 2 µl |
| 10 x Blunting Kination buffer | 2 µl |
| Blunting Kination Enzyme Mix | 1 µl |
| H₂O | 15 µl |
| | 20 µl |

After the transformation, colonies of *Escherichia coli* were checked by PCR (RE Inc., Ampli-Taq) with respect to the presence or absence and the direction of the insert (PCR product), and base sequences of three clones (#2, #4, #7) were checked, resulting that the base sequence of clone #7 was identical with the base sequence estimated to be the deleted region of N terminus on a data base.

For constructing the whole-length cDNA of a novel gene NZMP having a protease motif, the above clone #7 (pBS/hNZMP-N#7:having XbaI site of the fragment 3'-side and XbaI site of pBS vector on its 3'-side) was digested with XbaI, and then treated with BAP, and was ligated with a fragment (about 1.45k bp) obtained by digesting KIAA1226 (XbaI sites exist at the 184th from 5'-side and 288 bp downstream of the termination codon) with XbaI. Then, a competent cell (JM109) was transformed with the obtained nucleotide, and colonies of *Escherichia coli* were checked with PCR (using Ampli-Taq: RE Inc.) to construct pBS/hNZMP#1 containing the whole-length cDNA (Fig. 1).

### Example 2

### Expression of NZMP in Escherichia coli

In order to obtain NZMP, NZMP gene was expressed in *Escherichia coli*. An expression vector and an expression system for *E. coli* were constructed with a Gateway system (Life Technologies Inc.) according to the instruction of the Gateway cloning technology. The coding region of human NZMP was amplified using the primers described below, and using a cDNA as a template, which was obtained from a mRNA derived from the human brain by reverse-transcription with an oligo(dT) primer (refer to Example 1 with respect to the reverse transcription reaction and the PCR).

### Primers

Pr-DONRK1226-S01 (SEQ ID NO:5)

5′-GGGACAAGTTTGTACAAAAAAGCAGGCTACATGACGTTAGGAAGAGAAGTG-3′

Pr-DONRK1226-S02 (SEQ ID NO:6)

5′-GGGGACCACTTTGTACAAGAAAGCTGGGTCTTACGGAGCATGCAGGCCTCTAC-3′

Then, 45 µl of PCR product was diluted with TE buffer up to 200 µl, and 100 µl of 30% PEG8000 plus 30 mM MgCl₂ was added to centrifuge at 15,000 rpm at room temperature for 10 min. The pellet obtained was washed with 70% EtOH, dried, and dissolved in 10 µl of TE buffer. Then, 1 µl of PCR product, 0.5 µl of entry vector (pDONR201, 150 ng/µl), 1 µl of BP clonase reaction buffer, and 1.5 µl of TE buffer, were mixed on ice (total volume of 4 µl). After adding 1 µl of BP clonase enzyme mix, the reaction was performed at 25°C for 1 h. After the end of the reaction, 0.5 µl of Proteinase K was added to the mixture and incubated at 37°C for 10 min to inactivate the enzyme mix. A competent cell (DH5α) was transformed with 1 µl of the reaction mixture, and was subcloned, followed by checking the base sequence to obtain pDONR201/h-NZMP#1. The base sequence of the translation region of NZMP gene contained in the clone was determined by the common procedure. The obtained base sequence is shown as SEQ ID NO:2. The base sequence of NZMP contained in pDONR201/h-NZMP#1 was homologous to that of the database and that of KIAA1226 except for the 1908th nucleotide wherein 'A' was converted to 'G', which dose not lead to the amino acid substitution. Human NZMP consists of 681 amino acids (SEQ ID NO:1), and has a zinc metal peptidase motif from the 471st Thr to 480th Met.

Using this pDONR201/h-NZMP#1, a vector for expressing a fusion protein of NZMP with 6 x His tag was constructed. 1 µl of pDONR201/h-NZMP#1 (50 ng/µl), 6 x His tag expression vector (pDEST17:150 ng/µl), 1 µl of LR clonase reaction buffer, and 1.5 µl of TE buffer, were mixed on ice (total volume of 14 µl). Then 1 µl of LR clonase enzyme mix was added to the obtained mixture, followed by incubating at 25°C for 1 h. After adding 0.5 µl of Proteinase K to the reaction mixture, the enzyme mix was inactivated by incubating at 37°C for 10 min. A competent cell (BL21-SI, *Escherichia coli* with which expression is induced by NaCl) was transformed by 1 µl of the obtained reaction mixture, and colonies of *E. coli* were checked by PCR (using Ampli-Taq: RE Inc.), and three clones (pDEST17-hNZMP#1, pDEST17-hNZMP#3, pDEST17-hNZMP#5) were obtained.

With respect to the above three clones, the presence or absence of the induction of the NZMP production by NaCl and the water solubility of NZMP expressed, were tested.

Each clone of *Escherichia coli* was cultured in 2 ml of LB-Amp (NaCl-) with shaking at 37°C overnight. 600 µl of thus obtained pre-culture medium was transferred to 1.4 ml of LB-Amp (NaCl-), and was incubated with shaking at 37°C for 4 h. Then, 120 µl of 5M NaCl was added to give the final concentration of 0.3M, and the culture was incubated with shaking at 37°C for 2 h. For the negative control, 120 µl of H₂O was added, and the culture was incubated similarly. Then, the each culture medium was centrifuged at 3,000 rpm at 4°C for 10 min. The obtained pellet was resuspended with 200 µl of phosphate buffer saline (PBS) or 2% SDS plus 50 mM Tris (pH8.0), and was subjected to freezing and thawing, and to sonication, followed by centrifugation at 15,000 rpm at 4°C or 10°C for 10 min. The obtained supernatant was mixed with the same volume of 2 x Sample buffer [2% SDS/50 mM Tris (pH6.8) 30% glycerol/0.01%BPB] containing 2-ME (2-mercaptoethanol), and was boiled for 2 min. Then, SDS-PAGE was carried out using 8% polyacrylamide gel. As a result, as shown in Fig. 2, an about 75 kD band that is considered to be a fusion protein of human NZMP and 6 x His tag was detected. Among the clones thus obtained, plasmid pDEST17-hNZMP containing human NZMP gene kept by pDEST17-hNZMP#1 was deposited in National Institute of Bioscience and Human-Technology, Agency of Industrial Science and Technology, Ministry of International Trade and Industry as FERM BP-7269 on August 10, 2000.

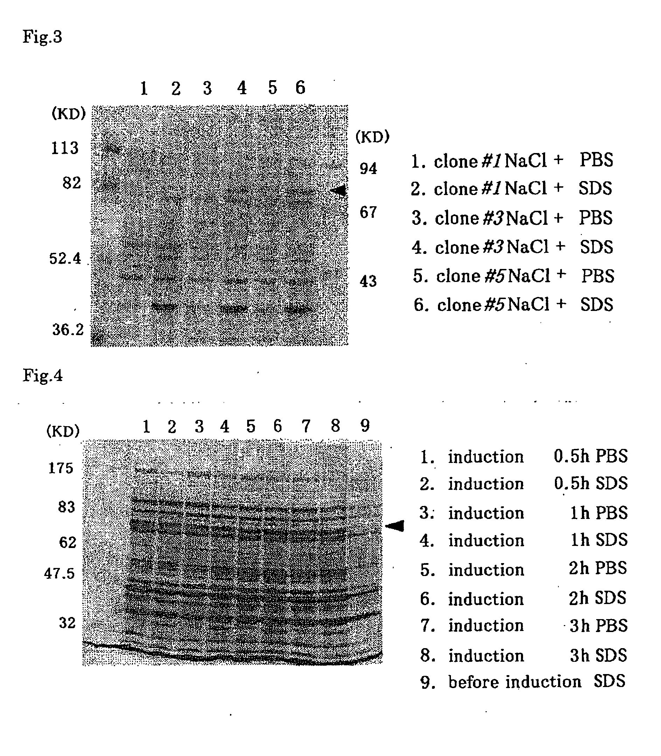

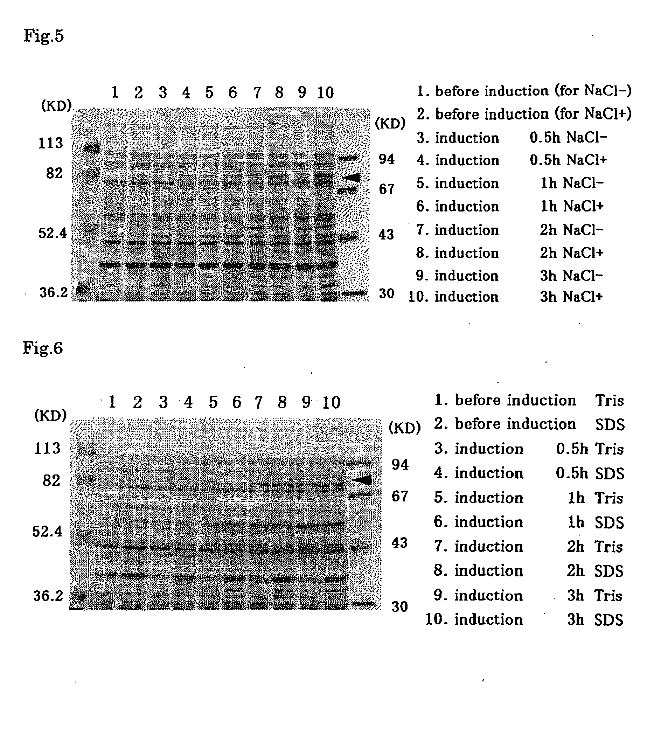

In addition, it was considered that a large amount of the gene product expressed as described above exists in an insoluble fraction (Fig. 3), so that culture conditions (culture temperature, OD₆₀₀ of *Escherichia coli* before induction, and induction time) were investigated for expressing NZMP as water-soluble one. As NZMP could be detected in a water-soluble fraction by culturing *E. coli* at as low as 30°C, *E. coli* was cultured in 2.5 ml of LB-Amp (NaCl-) with shaking at 30°C overnight. 2 ml of the obtained pre-culture medium was transferred to 18 ml of LB-Amp (NaCl-), and was incubated with shaking at 30°C for 2.5 h to give an OD₆₀₀ value of 1.0-2.0. Thereafter, in order to induce the production of NZMP, 5M NaCl was added to give the final concentration of 0.3M, and was incubated with shaking at 30°C for 3 h. For the negative control, H₂O was added, and the mixture was incubated in a similar way. Culture mediums were sampled before the induction and 0.5, 1, 2, and 3 h after the induction. Culture mediums were centrifuged at 15,000 rpm at 4°C for 10 min. The obtained pellet was resuspended in 100 µl of PBS and 2% SDS/50 mM Tris (pH8.0), and was treated by freezing and thawing, and by sonication, followed by centrifugation at 15,000 rpm at 4°C or 10°C for 10 min. The obtained supernatant was mixed with the same volume of 2 x sample buffer [2% SDS, 50 mM Tris (pH6.8), 30% glycerol, 0.01% BPB] containing 2-ME, and was boiled for 2 min. Then SDS-PAGE was carried out using 8% polyacrylamide gel. As illustrated in Fig. 4 and Fig. 5, it was found that appropriate conditions for obtaining water-soluble NZMP are a temperature of 30°C and an induction time for 2 to 3 h.

For preparing a specimen, the above *Escherichia coli* clone pDEST17-hNZMP#1 was cultured in 55 ml of LB-Amp (NaCl-) with shaking at 30°C overnight. 25 ml of the obtained pre-culture medium was transferred to 250 ml of LB-Amp (NaCl-) (to give a total of 275 ml), and was cultured with shaking at 30°C for 3 h 45 min to give an OD₆₀₀ value of 1.5-2.0. Thereafter, for inducing the NZMP production, 5M NaCl was added give the final concentration of 0.3M, and was incubated with shaking at 30°C for 3 h. For the negative control, H₂O was added, and the incubation was carried out in a similar way. 10 ml aliquots were sampled from the culture medium before the induction, and 0.5 h, 1 h, 2 h, and 3 h after the induction and were centrifuged at 3,000 rpm at 4°C for 10 min for harvesting cells. The obtained *E. coli* cells were suspended in 1 ml of 20 mM Tris (pH7.4) or 2% SDS plus 20 mM Tris (pH7.4), and was treated by freezing and thawing, and by sonication, followed by centrifugation at 15,000 rpm at 4°C or 10°C for 10min. The obtained supernatant was mixed with the same volume of 2 x sample buffer [2% SDS, 50 mM Tris (pH 6.8), 30% glycerol, 0.01% BPB] containing 2-ME, and was boiled for 2 min, followed by carrying out SDS-PAGE using 8% polyacrylamide gel. As a result, as illustrated in Fig. 6, water-soluble NZMP was obtained.

### Experiment 1

In order to investigate the enzyme activity of the obtained NZMP using a crude extract obtained by using 20 mM Tris (pH7.4) according to Example 2, total protein was measured quantitatively. As the substrate used for assaying the enzyme activity is a synthetic substrate consisting of 5 recognized residues and a protease activity existing in *Escherichia coli* is negligible to some extent, therefore it is not necessary to purify NZMP. For the quantitative analysis of the protein, a BCA assay kit was used (PIERCE Inc., the kit consists of reagent A and reagent B). A calibration curve was obtained using standard solutions that were prepared by 2-fold serial dilution of 2 mg/ml BSA aqueous solution (7 steps) with 20 mM Tris (pH7.4) to give a 1mg/ml solution and more diluted solutions. The extract solution was serially diluted from the original solution in a 2-fold manner (8 steps) with 20 mM Tris (pH7.4). The standard solutions and diluted extract solutions were added into a 96-well plate by 50 µl per well, and a reagent prepared by mixing reagent A and reagent B at a ratio of 50:1 was added by 200 µl per well, and then mixed to incubate at 37°C for 30 min, followed by measuring at OD₅₅₀. The total amount of protein in each extract was determined using the calibration curve.

Activity of the enzyme was assayed according to the method described in "Methods in Enzymology 248, 593-614, 1995" as follows: Each extract solution was diluted with 20 mM Tris (pH7.4) to give a total protein concentration of 200 µg/ml, and then diluted serially in a 2-fold manner (12 steps). 100 µl of each solution was added into each well of a 96-well plate. Synthetic substrate Mcc-Pro-Leu-Gly-Pro-D-Lys(DNP)-OH (Novabiochem Inc.) was dissolved into dimethylsulfoxide (DMSO) to give a concentration of 100 mM, and then 1,000-fold diluted with 20 mM Tris (pH7.4). The diluted substrate solution was added into each well by 100 µl, and was incubated at 37°C for 1 h. After the incubation, the fluorescence intensity was measured at an excitation wavelength of 345±10 nm and an emission wavelength of 425±10 nm.

For the control experiment, also with respect to *Escherichia coli* (BL21-SI) transformed with a vector (pDEST17-luciferase) for expressing luciferase that is an enzyme other than protease as a fusion protein with 6 x His tag, SDS-PAGE pattern and enzyme activity were investigated before the induction and 3 h after the induction.

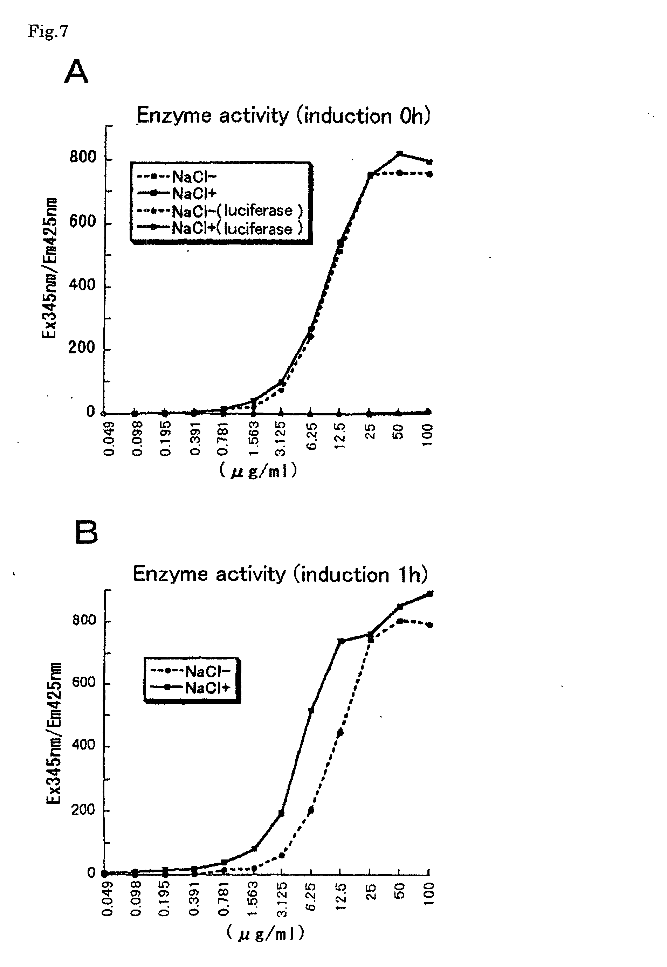

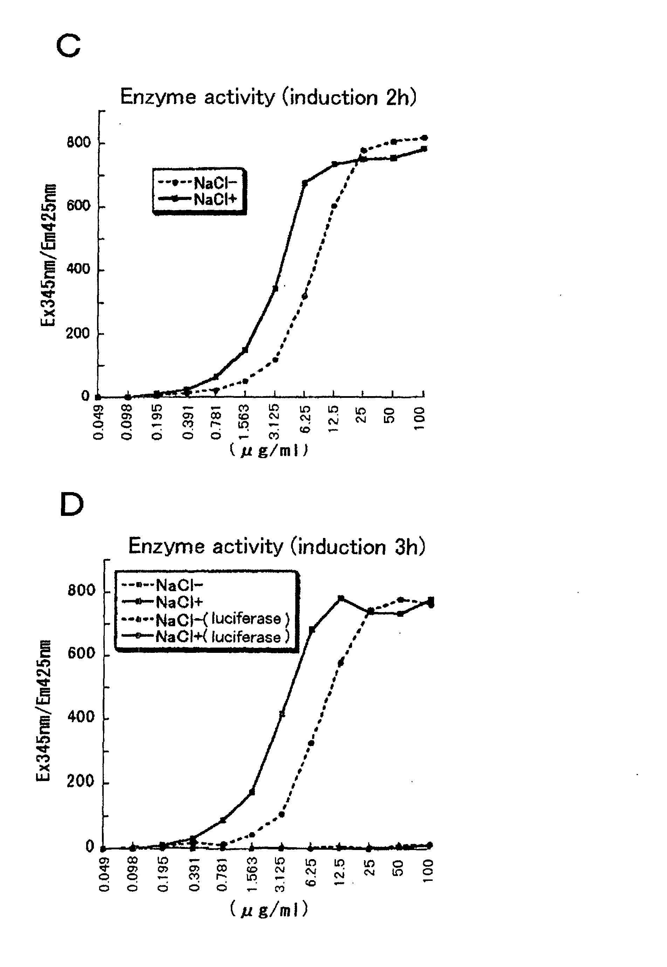

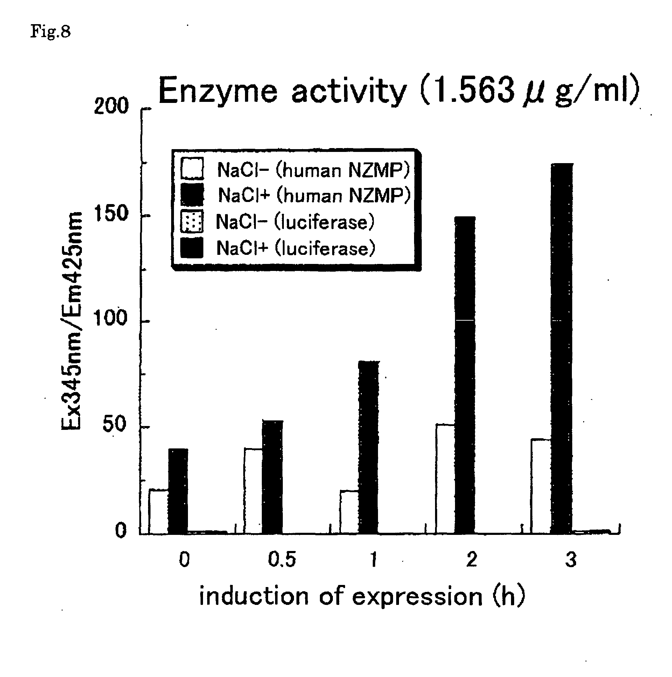

As a result, from the time when 1 h elapsed after the induction with NaCl, it was found that the enzyme activity, at a total protein concentration of 0.781 µg/ml to 12.5 µg/ml, was different with or without the induction by NaCl [Figs. 7A-7D]. The enzyme activity was enhanced by induction with NaCl (1, 2, 3 h) at a protein concentration of 1.563 µg/ml, while the enzyme activity was not detected in the water-soluble fraction in the case of a control in which luciferase gene was introduced into the similar expression vector or in which any vector was not introduced into *Escherichia coli* (Fig. 8). *E. coli* used as a control, into which luciferase gene was introduced, was confirmed to produce/induce luciferase.

From the above results, it was found that NZMP thus identified has an enzyme activity. In addition, it was showed that NZMP was expressed to some extent even without NaCl induction as illustrated in Fig. 8, and the expression of NZMP is enhanced by induction with NaCl.

### INDUSTRIAL APPLICABILITY

NZMP is a gene product of a novel protease gene identified based on KIAA1226, selected by using a protease motif as an indicator, from a cDNA library derived from the human brain stored in Kazusa DNA Research Institute, which is a zinc metal peptidase degrading synthetic substrate
Mcc-Pro-Leu-Gly-Pro-D-Lys(DNP)-OH. This gene is strongly expressed in the corpus callosum of human. The search for an natural intracerebral substrate based on the amino acid sequence of this synthetic substrate resulted in the discovery of dopamine D5 receptor as a candidate, suggesting that NZMP plays an important role in the neural transmission system in the brain. In addition, it was found that outside the brain, ICAM-1 has amino acid sequence of Pro-Leu-Gly-Pro at a binding site with LFA-1 that is a ligand of ICAM-1. Therefore, NZMP according to the present invention, a peptide derived from NZMP, and a nucleotide related with NZMP, are useful as a medicament for preventing/treating and/or a diagnostic means for diseases related to NZMP.

SEQUENCE LISTING

<110> DAIICHI PHARMACEUTICAL CO., LTD

<120> Novel protease gene

<130> GP01-1023

<140> PCT/JP01/07147
<141> 2001-08-21

<150> JP P2000-252503
<151> 2000-08-23

<160> 6

<170> PatentIn Ver. 2.1

<210> 1
<211> 681
<212> PRT
<213> Homo sapiens

<400> 1
Met Thr Leu Gly Arg Glu Val Met Ser Pro Leu Gln Ala Met Ser Ser
 1               5                  10                  15
Tyr Thr Val Ala Gly Arg Asn Val Leu Arg Trp Asp Leu Ser Pro Glu
            20                  25                  30
Gln Ile Lys Thr Arg Thr Glu Glu Leu Ile Val Gln Thr Lys Gln Val
        35                  40                  45
Tyr Asp Ala Val Gly Met Leu Gly Ile Glu Glu Val Thr Tyr Glu Asn
    50                  55                  60
Cys Leu Gln Ala Leu Ala Asp Val Glu Val Lys Tyr Ile Val Glu Arg
 65                  70                  75                  80
Thr Met Leu Asp Phe Pro Gln His Val Ser Ser Asp Lys Glu Val Arg
                 85                  90                  95
Ala Ala Ser Thr Glu Ala Asp Lys Arg Leu Ser Arg Phe Asp Ile Glu

            100                 105                 110
Met Ser Met Arg Gly Asp Ile Phe Glu Arg Ile Val His Leu Gln Glu
        115                 120                 125
Thr Cys Asp Leu Gly Lys Ile Lys Pro Glu Ala Arg Arg Tyr Leu Glu
    130                 135                 140
Lys Ser Ile Lys Met Gly Lys Arg Asn Gly Leu His Leu Pro Glu Gln
145                 150                 155                 160
Val Gln Asn Glu Ile Lys Ser Met Lys Lys Arg Met Ser Glu Leu Cys
                165                 170                 175
Ile Asp Phe Asn Lys Asn Leu Asn Glu Asp Asp Thr Phe Leu Val Phe
            180                 185                 190
Ser Lys Ala Glu Leu Gly Ala Leu Pro Asp Asp Phe Ile Asp Ser Leu
        195                 200                 205
Glu Lys Thr Asp Asp Asp Lys Tyr Lys Ile Thr Leu Lys Tyr Pro His
    210                 215                 220
Tyr Phe Pro Val Met Lys Lys Cys Cys Ile Pro Glu Thr Arg Arg Arg
225                 230                 235                 240
Met Glu Met Ala Phe Asn Thr Arg Cys Lys Glu Glu Asn Thr Ile Ile
                245                 250                 255
Leu Gln Gln Leu Leu Pro Leu Arg Thr Lys Val Ala Lys Leu Leu Gly
            260                 265                 270
Tyr Ser Thr His Ala Asp Phe Val Leu Glu Met Asn Thr Ala Lys Ser
        275                 280                 285
Thr Ser Arg Val Thr Ala Phe Leu Asp Asp Leu Ser Gln Lys Leu Lys
    290                 295                 300
Pro Leu Gly Glu Ala Glu Arg Glu Phe Ile Leu Asn Leu Lys Lys Lys
305                 310                 315                 320
Glu Cys Lys Asp Arg Gly Phe Glu Tyr Asp Gly Lys Ile Asn Ala Trp
                325                 330                 335
Asp Leu Tyr Tyr Tyr Met Thr Gln Thr Glu Glu Leu Lys Tyr Ser Ile
            340                 345                 350
Asp Gln Glu Phe Leu Lys Glu Tyr Phe Pro Ile Glu Val Val Thr Glu
        355                 360                 365
Gly Leu Leu Asn Thr Tyr Gln Glu Leu Leu Gly Leu Ser Phe Glu Gln
    370                 375                 380
Met Thr Asp Ala His Val Trp Asn Lys Ser Val Thr Leu Tyr Thr Val
385                 390                 395                 400

Lys Asp Lys Ala Thr Gly Glu Val Leu Gly Gln Phe Tyr Leu Asp Leu
                405                 410                 415
Tyr Pro Arg Glu Gly Lys Tyr Asn His Ala Ala Cys Phe Gly Leu Gln
            420                 425                 430
Pro Gly Cys Leu Leu Pro Asp Gly Ser Arg Met Met Ala Val Ala Ala
        435                 440                 445
Leu Val Val Asn Phe Ser Gln Pro Val Ala Gly Arg Pro Ser Leu Leu
    450                 455                 460
Arg His Asp Glu Val Arg Thr Tyr Phe His Glu Phe Gly His Val Met
465                 470                 475                 480
His Gln Ile Cys Ala Gln Thr Asp Phe Ala Arg Phe Ser Gly Thr Asn
                485                 490                 495
Val Glu Thr Asp Phe Val Glu Val Pro Ser Gln Met Leu Glu Asn Trp
            500                 505                 510
Val Trp Asp Val Asp Ser Leu Arg Arg Leu Ser Lys His Tyr Lys Asp
        515                 520                 525
Gly Ser Pro Ile Ala Asp Asp Leu Leu Glu Lys Leu Val Ala Ser Arg
    530                 535                 540
Leu Val Asn Thr Gly Leu Leu Thr Leu Arg Gln Ile Val Leu Ser Lys
545                 550                 555                 560
Val Asp Gln Ser Leu His Thr Asn Thr Ser Leu Asp Ala Ala Ser Glu
                565                 570                 575
Tyr Ala Lys Tyr Cys Ser Glu Ile Leu Gly Val Ala Ala Thr Pro Gly
            580                 585                 590
Thr Asn Met Pro Ala Thr Phe Gly His Leu Ala Gly Gly Tyr Asp Gly
        595                 600                 605
Gln Tyr Tyr Gly Tyr Leu Trp Ser Glu Val Phe Ser Met Asp Met Phe
    610                 615                 620
Tyr Ser Cys Phe Lys Lys Glu Gly Ile Met Asn Pro Glu Val Gly Met
625                 630                 635                 640
Lys Tyr Arg Asn Leu Ile Leu Lys Pro Gly Gly Ser Leu Asp Gly Met
                645                 650                 655
Asp Met Leu His Asn Phe Leu Lys Arg Glu Pro Asn Gln Lys Ala Phe
            660                 665                 670
Leu Met Ser Arg Gly Leu His Ala Pro
        675                 680

<210> 2
<211> 2046
<212> DNA
<213> Homo sapiens

<220>
<221> CDS
<222> (1)..(2046)

<400> 2
atg acg tta gga aga gaa gtg atg tct cct ctt cag gca atg tct tcc    48
Met Thr Leu Gly Arg Glu Val Met Ser Pro Leu Gln Ala Met Ser Ser
1               5                   10                  15

tat act gtg gct ggc aga aat gtt tta aga tgg gat ctt tca cca gag    96
Tyr Thr Val Ala Gly Arg Asn Val Leu Arg Trp Asp Leu Ser Pro Glu
            20                  25                  30

caa att aaa aca aga act gag gag ctc att gtg cag acc aaa cag gtg    144
Gln Ile Lys Thr Arg Thr Glu Glu Leu Ile Val Gln Thr Lys Gln Val
        35                  40                  45

tac gat gct gtt gga atg ctc ggt att gag gaa gta act tac gag aac    192
Tyr Asp Ala Val Gly Met Leu Gly Ile Glu Glu Val Thr Tyr Glu Asn
    50                  55                  60

tgt ctg cag gca ctg gca gat gta gaa gta aag tat ata gtg gaa agg    240
Cys Leu Gln Ala Leu Ala Asp Val Glu Val Lys Tyr Ile Val Glu Arg
65                  70                  75                  80

acc atg cta gac ttt ccc cag cat gta tcc tct gac aaa gaa gta cga    288
Thr Met Leu Asp Phe Pro Gln His Val Ser Ser Asp Lys Glu Val Arg
                85                  90                  95

gca gca agt aca gaa gca gac aaa aga ctt tct cgt ttt gat att gag    336
Ala Ala Ser Thr Glu Ala Asp Lys Arg Leu Ser Arg Phe Asp Ile Glu

            100                 105                 110

atg agc atg aga gga gat ata ttt gag aga att gtt cat tta cag gaa 384
Met Ser Met Arg Gly Asp Ile Phe Glu Arg Ile Val His Leu Gln Glu
        115                 120                 125

acc tgt gat ctg ggg aag ata aaa cct gag gcc aga cga tac ttg gaa 432
Thr Cys Asp Leu Gly Lys Ile Lys Pro Glu Ala Arg Arg Tyr Leu Glu
    130                 135                 140

aag tca att aaa atg ggg aaa aga aat ggg ctc cat ctt cct gaa caa 480
Lys Ser Ile Lys Met Gly Lys Arg Asn Gly Leu His Leu Pro Glu Gln
145                 150                 155                 160

gta cag aat gaa atc aaa tca atg aag aaa aga atg agt gag cta tgt 528
Val Gln Asn Glu Ile Lys Ser Met Lys Lys Arg Met Ser Glu Leu Cys
                165                 170                 175

att gat ttt aac aaa aac ctc aat gag gat gat acc ttc ctt gta ttt 576
Ile Asp Phe Asn Lys Asn Leu Asn Glu Asp Asp Thr Phe Leu Val Phe
            180                 185                 190

tcc aag gct gaa ctt ggt gct ctt cct gat gat ttc att gac agt tta 624
Ser Lys Ala Glu Leu Gly Ala Leu Pro Asp Asp Phe Ile Asp Ser Leu
        195                 200                 205

gaa aag aca gat gat gac aag tat aaa att acc tta aaa tat cca cac 672
Glu Lys Thr Asp Asp Asp Lys Tyr Lys Ile Thr Leu Lys Tyr Pro His
    210                 215                 220

tat ttc cct gtc atg aag aaa tgt tgt atc cct gaa acc aga aga agg 720
Tyr Phe Pro Val Met Lys Lys Cys Cys Ile Pro Glu Thr Arg Arg Arg
225                 230                 235                 240

atg gaa atg gct ttt aat aca agg tgc aaa gag gaa aac acc ata att 768
Met Glu Met Ala Phe Asn Thr Arg Cys Lys Glu Glu Asn Thr Ile Ile
                245                 250                 255

ttg cag cag cta ctc cca ctg cga acc aag gtg gcc aaa cta ctc ggt 816
Leu Gln Gln Leu Leu Pro Leu Arg Thr Lys Val Ala Lys Leu Leu Gly
            260                 265                 270

tat agc aca cat gct gac ttc gtc ctt gaa atg aac act gca aag agc 864
Tyr Ser Thr His Ala Asp Phe Val Leu Glu Met Asn Thr Ala Lys Ser
        275                 280                 285

aca agc cgc gta aca gcc ttt cta gat gat tta agc cag aag tta aaa 912
Thr Ser Arg Val Thr Ala Phe Leu Asp Asp Leu Ser Gln Lys Leu Lys
    290                 295                 300

ccc ttg ggt gaa gca gaa cga gag ttt att ttg aat ttg aag aaa aag 960
Pro Leu Gly Glu Ala Glu Arg Glu Phe Ile Leu Asn Leu Lys Lys Lys
305                 310                 315                 320

gaa tgc aaa gac agg ggt ttt gaa tat gat ggg aaa atc aat gcc tgg 1008
Glu Cys Lys Asp Arg Gly Phe Glu Tyr Asp Gly Lys Ile Asn Ala Trp
                325                 330                 335

gat cta tat tac tac atg act cag aca gag gaa ctc aag tat tcc ata 1056
Asp Leu Tyr Tyr Tyr Met Thr Gln Thr Glu Glu Leu Lys Tyr Ser Ile
            340                 345                 350

gac caa gag ttc ctc aag gaa tac ttc cca att gag gtg gtc act gaa 1104
Asp Gln Glu Phe Leu Lys Glu Tyr Phe Pro Ile Glu Val Val Thr Glu
        355                 360                 365

ggc ttg ctg aac acc tac cag gag ttg ttg gga ctt tca ttt gaa caa 1152
Gly Leu Leu Asn Thr Tyr Gln Glu Leu Leu Gly Leu Ser Phe Glu Gln
    370                 375                 380

atg aca gat gct cat gtt tgg aac aag agt gtt aca ctt tat act gtg 1200
Met Thr Asp Ala His Val Trp Asn Lys Ser Val Thr Leu Tyr Thr Val
385                 390                 395                 400

aag gat aaa gct aca gga gaa gta ttg gga cag ttc tat ttg gac ctc 1248
Lys Asp Lys Ala Thr Gly Glu Val Leu Gly Gln Phe Tyr Leu Asp Leu
                405                 410                 415

tat cca agg gaa gga aaa tac aat cat gcg gcc tgc ttc ggt ctc cag 1296
Tyr Pro Arg Glu Gly Lys Tyr Asn His Ala Ala Cys Phe Gly Leu Gln
            420                 425                 430

cct ggc tgc ctt ctg cct gat gga agc cgg atg atg gca gtg gct gcc 1344
Pro Gly Cys Leu Leu Pro Asp Gly Ser Arg Met Met Ala Val Ala Ala
        435                 440                 445

ctc gtg gtg aac ttc tca cag cca gtg gca ggt cgt ccc tct ctc ctg 1392
Leu Val Val Asn Phe Ser Gln Pro Val Ala Gly Arg Pro Ser Leu Leu
    450                 455                 460

aga cac gac gag gtg agg act tac ttt cat gag ttt ggt cac gtg atg 1440
Arg His Asp Glu Val Arg Thr Tyr Phe His Glu Phe Gly His Val Met
465                 470                 475                 480

cat cag att tgt gca cag act gat ttt gca cga ttt agc gga aca aat 1488
His Gln Ile Cys Ala Gln Thr Asp Phe Ala Arg Phe Ser Gly Thr Asn
                485                 490                 495

gtg gaa act gac ttt gta gag gtg cca tcg caa atg ctt gaa aat tgg 1536
Val Glu Thr Asp Phe Val Glu Val Pro Ser Gln Met Leu Glu Asn Trp
            500                 505                 510

gtg tgg gac gtc gat tcc ctc cga aga ttg tca aaa cat tat aaa gat 1584
Val Trp Asp Val Asp Ser Leu Arg Arg Leu Ser Lys His Tyr Lys Asp
        515                 520                 525

gga agc cct att gca gac gat ctg ctt gaa aaa ctt gtt gct tct agg 1632
Gly Ser Pro Ile Ala Asp Asp Leu Leu Glu Lys Leu Val Ala Ser Arg
    530                 535                 540

ctg gtc aac aca ggt ctt ctg acc ctg cgc cag att gtt ttg agc aaa 1680

Leu Val Asn Thr Gly Leu Leu Thr Leu Arg Gln Ile Val Leu Ser Lys
545                 550                 555                 560

gtt gat cag tct ctt cat acc aac aca tcg ctg gat gct gca agt gaa     1728
Val Asp Gln Ser Leu His Thr Asn Thr Ser Leu Asp Ala Ala Ser Glu
                565                 570                 575

tat gcc aaa tac tgc tca gaa ata tta gga gtt gca gct act cca ggc     1776
Tyr Ala Lys Tyr Cys Ser Glu Ile Leu Gly Val Ala Ala Thr Pro Gly
            580                 585                 590

aca aat atg cca gct acc ttt gga cat ttg gca ggg gga tac gat ggc     1824
Thr Asn Met Pro Ala Thr Phe Gly His Leu Ala Gly Gly Tyr Asp Gly
        595                 600                 605

caa tat tat gga tat ctt tgg agt gaa gta ttt tcc atg gat atg ttt     1872
Gln Tyr Tyr Gly Tyr Leu Trp Ser Glu Val Phe Ser Met Asp Met Phe
    610                 615                 620

tac agc tgt ttt aaa aaa gaa ggg ata atg aat ccg gag gtt gga atg     1920
Tyr Ser Cys Phe Lys Lys Glu Gly Ile Met Asn Pro Glu Val Gly Met
625                 630                 635                 640

aaa tac aga aac cta atc ctg aaa cct ggg gga tct ctg gac ggc atg     1968
Lys Tyr Arg Asn Leu Ile Leu Lys Pro Gly Gly Ser Leu Asp Gly Met
                645                 650                 655

gac atg ctc cac aat ttc ttg aaa cgt gag cca aac caa aaa gcg ttc     2016
Asp Met Leu His Asn Phe Leu Lys Arg Glu Pro Asn Gln Lys Ala Phe
            660                 665                 670

cta atg agt aga ggc ctg cat gct ccg taa                             2046
Leu Met Ser Arg Gly Leu His Ala Pro
        675                 680

<210> 3

<211> 26
<212> DNA
<213> Artificial Sequence

<220>
<223> Description of Artificial Sequence:Designed
      oligonucleotide used for PCR primer

<400> 3
aggtcgacat gacgttagga agagaa                                          26


<210> 4
<211> 22
<212> DNA
<213> Artificial Sequence

<220>
<223> Description of Artificial Sequence:Designed
      oligonucleotide used for PCR primer

<400> 4
tcatctagaa aggctgttac gc                                              22


<210> 5
<211> 51
<212> DNA
<213> Artificial Sequence

<220>
<223> Description of Artificial Sequence:Designed
      oligonuclotide used for PCR primer

<400> 5
gggacaagtt tgtacaaaaa agcaggctac atgacgttag gaagagaagt g              51

<210> 6
<211> 53
<212> DNA
<213> Artificial Sequence

<220>
<223> Description of Artificial Sequence:Designed
      oligonucleotide used for PCR primer

<400> 6
ggggaccact ttgtacaaga aagctgggtc ttacggagca tgcaggcctc tac          53

Sequence table free text
SEQ ID NO:3: Designed base sequence used for PCR primer
SEQ ID NO:4: Designed base sequence used for PCR primer
SEQ ID NO:5: Designed base sequence used for PCR primer
SEQ ID NO:6: Designed base sequence used for PCR primer

## Claims

1. a peptide selected from a group consisting of:
(i) a peptide consisting of the amino acid sequence of SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH, and
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH.

2. A peptide having an amino acid sequence that consists of four consecutive amino acids or more of the amino acid sequence of SEQ ID NO:1.

3. A polynucleotide encoding a peptide selected from a group consisting of the following peptides, and a complementary strand thereto:
(i) a peptide consisting of the amino acid sequence of SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH, and
(v) a peptide having an amino acid sequence that consists of four consecutive amino acids or more of the amino acid sequence of SEQ ID NO:1.

4. A polynucleotide consisting of the base sequence of SEQ ID NO:2 or a complementary strand thereto.

5. A polynucleotide according to claim 3 or 4, wherein the polynucleotide hybridizes under stringent conditions.

6. A polynucleotide consisting of a base sequence that consists of at least twelve consecutive bases of the base sequence of the polynucleotide encoding the amino acid sequence of SEQ ID NO:1 or a complementary strand thereto.

7. A recombinant vector comprising a polynucleotide encoding a peptide selected from the group consisting of those from (i) to (v) or a polynucleotide selected from a group consisting of those from (vi) to (viii):
(i) a peptide consisting of the amino acid sequence of SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
(v) a peptide having an amino acid sequence that consists of four consecutive amino acids or more of the amino acid sequence of SEQ ID NO:1,
(vi) a polynucleotide consisting of the base sequence of SEQ ID NO:2, or a complementary strand thereto,
(vii) a polynucleotide that hybridizes to a polynucleotide encoding the peptide described in (i), (ii), (iii), (iv) or (v) under stringent conditions, and
(viii) a polynucleotide consisting of a base sequence that consists of at least twelve consecutive bases of the base sequence of the polynucleotide encoding the amino acid sequence of SEQ ID NO:1 or a complementary strand thereto.

8. Plasmid pDEST17-hNZMP (FERM BP-7269).

9. A transformant transformed with a recombinant vector comprising a polynucleotide encoding a peptide selected from the group consisting of those from(i) to (v), a recombinant vector comprising a polynucleotide selected from the group consisting of those from (vi) to (viii), or the plasmid (ix):
(i) a peptide consisting of the amino acid sequence of SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
(v) a peptide having an amino acid sequence that consists of four consecutive amino acids or more of the amino acid sequence of SEQ ID NO:1,
(vi) a polynucleotide consisting of the base sequence of SEQ ID NO:2 or a complementary strand thereto,
(vii) a polynucleotide that hybridizes to a polynucleotide encoding the peptide described in (i), (ii), (iii), (iv) or (v) under stringent conditions,
(viii) a polynucleotide consisting of a base sequence that consists of at least twelve consecutive bases of the base sequence of the polynucleotide encoding the amino acid sequence of SEQ ID NO:1 or a complementary strand thereto, and
(ix) Plasmid pDEST17-hNZMP (FERM BP-7269).

10. A method for producing a peptide of claim 1 or 2, comprising a step of culturing a transformant of claim 9.

11. An antibody that immunologically recognizes a peptide selected from a group consisting of:
(i) a peptide consisting of the amino acid sequence of SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH, and
(v) a peptide having an amino acid sequence that consists of four consecutive amino acids or more of the amino acid sequence of SEQ ID NO:1.

12. An antibody of claim 11, wherein the antibody has at least a function to decrease the activity of degrading synthetic substrate Mcc-Pro-Leu-Gly-Pro-D-Lys(DNP)-OH.

13. A method for identifying a compound that interacts with a peptide selected from a group consisting from (i) to (v) to decrease or increase its activity, and/or a compound that interacts with a polynucleotide selected from a group consisting from (vi) to (viii) to decrease or increase its expression, wherein the method uses at least one selected from a group consisting of the peptide, the polynucleotide, a recombinant vector comprising the polynucleotide, plasmid pDEST17-hNZMP (FERM BP-7269), a transformant obtained by using the recombinant vector or the plasmid, and an antibody that immunologically recognizes the peptide:
(i) a peptide consisting of the amino acid sequence show by the sequence listing,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
(v) a peptide having an amino acid sequence that consists of four consecutive amino acids or more of the amino acid sequence of SEQ ID NO:1,
(vi) a polynucleotide consisting of a base sequence of SEQ ID NO:2,
(vii) a polynucleotide encoding each of the peptides described in (i)-(v) or a polynucleotide that hybridizes to the polynucleotide under stringent conditions, and
(viii) a polynucleotide consisting of a base sequence that consists of at least twelve consecutive bases of the base sequence of the polynucleotide encoding the amino acid sequence of SEQ ID NO:1 or a complementary strand thereto.

14. a method for identifying a compound according to claim 13, wherein the method includes contacting a peptide with a compound to be screened under conditions that permit the interaction of the compound with the peptide, evaluating the interaction of the compound, and detecting a signal given by the interaction of the compound with the peptide, followed by determining whether the compound increases or decreases the activity by the interaction of the compound with the peptide.

15. A method for identifying a compound according to claim 13 or 14, wherein the method includes contacting a transformant with a compound to be screened, introducing a system using a signal and/or marker capable of detecting the expression of the peptide, detecting the presence of the signal, followed by determining the presence or absence of an increase or decrease of the expression of the peptide.

16. A compound identified by a method according to any of claims 13 to 15.

17. A compound of claim 16, which interacts with the peptide to decrease or increase its activity.

18. A compound of claim 16, which interacts with the polynucleotide to decrease or increase its expression.

19. A pharmaceutical composition comprising any one selected from the group consisting of the following peptides (i)-(v), the following polynucleotides (vi)-(viii), recombinant vectors containing the polynucleotides, plasmid pDEST17-hNZMP (FERM BP-7269), transformants obtained by using the recombinant vectors or the plasmids, and antibodies that immunologically recognize the peptides:
(i) a peptide consisting of the amino acid sequence show by SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
(v) a peptide having an amino acid sequence that consists of four consecutive amino acids or more of the amino acid sequence of SEQ ID NO:1,
(vi) a polynucleotide consisting of the base sequence of SEQ ID NO:2,
(vii) a polynucleotide encoding each of the peptides described in (i)-(v) or a polynucleotide that hybridizes to the polynucleotide under stringent conditions, and
(viii) a polynucleotide consisting of a base sequence that consists of at least twelve consecutive bases of the base sequence of the polynucleotide encoding the amino acid sequence of SEQ ID NO:1 or a complementary strand thereto.

20. A use of the pharmaceutical composition of claim 19 as a medicament for preventing/treating diseases that relates to the peptide consisting of an amino acid sequence of SEQ ID NO:1.

21. A method for preventing/treating diseases that relates to the peptide consisting of an amino acid sequence of SEQ ID NO:1, using the pharmaceutical composition of claim 19.

22. A method for diagnosing diseases that relate to the expression or activity of a peptide selected from a group consisting of the following peptides (i)-(v), comprising analyzing for a peptide selected from a group consisting of the following peptides (i)-(v) or a polynucleotide selected from the group consisting of the following polynucleotides (vi)-(viii) as a marker:
(i) a peptide consisting of the amino acid sequence show by SEQ ID NO:1,
(ii) a peptide comprising the peptide described in (i),
(iii) a peptide that has an amino acid sequence homologous to the peptide described in (i) at about 40% or more, and has an activity of degrading synthetic substrate
Mcc-Pro-Leu-Gly-Pro-D-Lys(DNP)-OH,
(iv) a peptide modified by mutation such as deletion, substitution, addition or insertion of one or several amino acids in the peptide described in (i), (ii) or (iii), and has an activity of degrading synthetic substrate Mcc-Pro-Leu-Gly-Pro-D-Lys(DNP)-OH,
(v) a peptide having an amino acid sequence that consists of four consecutive amino acids or more of the amino acid sequence of SEQ ID NO:1,
(vi) a polynucleotide consisting of the base sequence of SEQ ID NO:2,
(vii) a polynucleotide encoding each of the peptides described in (i)-(v) or a polynucleotide that hybridizes to the polynucleotide under stringent conditions, and
(viii) a polynucleotide consisting of a base sequence that consists of at least twelve consecutive bases of the base sequence of the polynucleotide encoding the amino acid sequence of SEQ ID NO:1 or a complementary strand thereto.
